# EUROPEAN PATENT SPECIFICATION

(11) **EP 2 340 016 B1**
(45) Date of publication and mention of the grant of the patent: **23.01.2013**
(21) Application number: 09753062.0
(22) Date of filing: 29.10.2009
(51) Int. Cl.: A61K 9/06, A61K 47/34, A61K 31/205, A61P 17/00

(54) **TOPICAL FORMULATION OF 3-(2,2,2-TRIMETHYLHYDRAZINIUM) PROPIONATE DIHYDRATE**
TOPISCHE FORMULIERUNG VON 3-(2,2,2-TRIMETHYLHYDRAZINIUM)PROPIONATDIHYDRAT
FORMULE TOPIQUE DE DIHYDRATE PROPIONATE 3-(2,2,2-TRIMETHYLHYDRAZINIUM)

(30) Priority: 29.10.2008 EP 08167825
(43) Date of publication of application: 06.07.2011
(73) Proprietor: Grindeks, A Joint Stock Company, 1057 Riga (LV)
(72) Inventor: GEORGI, Malimon, LV-1057 Riga (LV)
(86) International application number: PCT/EP2009/064285
(87) International publication number: WO 2010/049485

(56) References cited:
- WO-A-2005/012233
- WO-A-2006/022536

## Description

### Technical field

The invention relates to a topical gel formulation of 3-(2,2,2-trimethylhydrazinium) propionate dihydrate for treatment of tissue and skin irritation.

### Background art

The healing of tissue lesions is a natural restorative response to tissue injury. Healing is the interaction of a complex cascade of cellular events that generates resurfacing, reconstitution, and restoration of the tensile strength of injured skin. Healing is a systematic process, traditionally explained in terms of 3 classic phases: inflammation, proliferation and maturation.

Therefore as further object of this invention was to provide for an agent to treat tissue lesions characterized by stable formulation. As active ingredient 3-(2,2,2-trimethylhydrazinium) propionate dihydrate was used for topical gel formulation.

Active ingredient 3-(2,2,2-trimethylhydrazinium) propionate dihydrate is disclosed in WO 8001068 (ORCH SINTEZA; EREMEEV; LATVIETIS; GILLER; TRAPENTSIERS; SEMENIKHI) 29.05.1980 and it is well known that its International Nonproprietary Name is Meldonium dihydrate (registered with the trade mark of "MILDRONA̅TS®", "MILDRONATE®").

Meldonium dihydrate was designed as an inhibitor of carnitine biosynthesis aimed to prevent accumulation of cytotoxic intermediate products of fatty acid β-oxidation in ischemic tissues and to block this highly oxygen-consuming process. Alternatively the Meldonium dihydrate acts via stimulation of the nitric oxide production in the vascular endothelium through a modification of the γ-butyrobetaine and γ-butyrobetaine ester pools.

The patent WO 2006022536 (JOINT STOCK COMPANY GRINDEKS; KALVINS IVARS; BIRMANS ANATOLIJS ) 02.03.2006 disclosed gel formulation of 3-(2,2,2-trimethylhydrazinium) propionate dihydrate for topical administration that comprise variable concentrations of natural and synthetic steroid and non-steroid antiinflammatory agents, analgesics and anaesthetics, antifungal medicines, antibiotics, antivirals, antihistamine drugs, enzymes, co-enzyms, dermatological drugs, medicines for treating tissue lesions, bees products. The medical use of the gel composition, which is disclosed in this patent, is for treating and/or preventing tissue lesions of skin and subcutaneous. Nevertheless shelf life of this gel is unsatisfactory.

For preparing ointments, creams, and the like, typically from 0.1 to 20 %, in particular from 1 to 15% and more in particular from 5 to 15% of active ingredient Meldonium dihydrate, is combined in intimate admixture with a skin-acceptable carrier.

In ointments or creams the carrier consists of 1-20%, in particular 1 to 5 % of humectants, in particular from 0.1 to 2% of a thickener and water and in particular from 0.1 to 1 % of preservatives.

Suitable humectants include propylene glycol, suitable thickening agent include for instance Carbomer such as Carbomer Carbopol 934, 980, 981, 1382, 5984, 2984 and as suitable preservatives are included methyl parahydroxybenzoate and ethyl parahydroxybenzoate.

It is known what propylene glycol is an organic molecule with two alcohol groups and is used as humectants. It is the most common moisture-carrying vehicle other than water itself and helps prevent moisture loss in products as well as binding to moisture and holding it to the skin. Being a large organic alcohol, propylene glycol provides excellent moisturizing properties to the skin.

Methyl parahydroxybenzoate and ethyl parahydroxybenzoate are known as methylparaben and ethylparaben and are used as preservatives. These preservatives were developed in the 1930's to stabilize creams. It is known that methylparaben is found naturally in plant sources from the fruit of the blueberry shrub. Chemically, parabens are esters of para-hydroxybenzoic acid, from which the name is derived. Typically, more than one paraben is used in a product to provide a broader antiseptic action and preservation against a broad range of microorganisms. The use of mixtures of parabens allows the use of lower levels while increasing preservative activity. Carbopol®-type resins, such as Carbopol® (Carbopol 934, 980, 981, 1382, 5984, 2984), Pemulen®, Noveon®, are polymers of acrylic acid, crosslinked with polyalkenyl ethers or divinyl glycocol.

Carbopol® 980 NF is obtained from Noveon (Cleveland, Ohio). Carbopol® 980 NF polymers are a highly efficient thickener and it is ideal for formulating clear aqueous and hydroalcoholic gels. Addition of a small amount of surfactant or neutralizer can alter the polymer conformation and the viscosity of the dispersion. Neutralizer works primarily by forming ionpairs. The surfactants, on the other hand, act through hydrophobic interaction between the nonpolar surfactant tail and the polymer backbone or solvent, and through electrostatic interactions between the polar beads of the surfactant and the charged group of the polymers. Depending on the choice of the polymer and solvent, one or both mechanisms are responsible for gel formation. When the polymers uncoil because of the formation of hydrogen bonds or electrostatic interaction, the gel structure is formed. Different solvent systems are used to satisfy compatibility requirements with active drugs and/or other excipients, and to ensure bioavailability of the drug when applied on skin.

Polymer-solvent-neutralizer and polymer-solvent-surfactant interactions modify the viscosity, elasticity, and other important properties, and contribute to the stability of the gels. These interactions can significantly affect the medicament diffusion from the gel to skin for transdermal delivery of drugs. This is especially important in case of topical gels because the interaction may change during solvent evaporation when the gel is applied on skin. Also the bioavailability of drugs changes owing to solvent evaporation.

### Disclosure of the invention

### Technical problem

As it's well-known Meldonium as dihydrate has essential drawbacks, the first of which consists in its rather high hygroscopicity. Already after 24 hours maintenance at 100% air humidity, mass of Meldonium dihydrate is increased by 10 % because of water absorption, the substance being transformed into syrup.

Besides, Meldonium dihydrate is not very stable: while heated, it fast loses the water of crystal hydrate. In turn, the anhydrous form of Meldonium dihydrate is unstable and extremely hygroscopic. In such form, this compound soon becomes coloured and gets a specific annoying odour. Thus, the hygroscopicity and thermal non-stability of Meldonium dihydrate are significant disadvantages. Furthermore, Meldonium dihydrate is actively dehydrated at temperatures as low as 40-45°C. This means that sure storage of Meldonium dihydrate dosage forms containing crystal hydrate is rather embarrassing in countries with hot climate.

The topical gel formulation of Meldonium dihydrate is described in WO 2006022536 (JOINT STOCK COMPANY GRINDEKS; KALVINS IVARS; BIRMANS ANATOLIJS ) 02.03.2006 , as exciepients is used- thickening agent sodium starch glycollate type C, as preservatives are used methyl parahydroxybenzoate and ethyl parahydroxybenzoate, as humectant is used propylene glycol and fumaric acid, but this topical gel formulation has a problem with shell life. The same exciepients were used for preparation gel there active ingredient was Meldonium dihydrate salts, the formulation disclosed in WO 2005012233 (GRINDEKS PUBLIC JOINT STOCK CO; KALVINSH IVARS; BIRMANS ANATOLIJS) 10.02.2005.

### Technical solution

By present invention surprisingly discovered that by using as exciepients for Meldonium dihydrate topical gel as thickening agent is Carbopol 980, as preservatives are used methyl parahydroxybenzoate and ethyl parahydroxybenzoate, as humectants is used propylene glycol and increasing pH of topical gel with sodium hydroxide the shell life of Meldonium dihydrate topical gel is increased.

### Advantageous effects

The main and the most important advantage is what Meldonium dihydrate topical gel has a stable formulation and shelf life is increased.

### Best mode for carrying out the invention

### Comparative tests

Comparative tests were provided by using Meldonium dihydrate gel formulation which were disclosed in WO 2006022536 (JOINT STOCK COMPANY GRINDEKS; KALVINS IVARS; BIRMANS ANATOLIJS) 02.03.2006 and in WO 2005012233 (GRINDEKS PUBLIC JOINT STOCK CO; KALVINSH IVARS; BIRMANS ANATOLIJS) 10.02.2005 which correspond to the Meldonium gel formulation A in below Table 1. Accordingly Meldonium dihydrate gel formulation B, C, D, E, F and G refers to use of different kind of Carbomers as thickeners.

**Table 1**

| Ingredient | Meldonium dihydrate gel formulation, % w/w | | | | | | |
|---|---|---|---|---|---|---|---|
| | A | B | C | D | E | F | G |
| Meldonium dihydrate | 10 | 10 | 10 | 10 | 10 | 10 | 10 |
| Sodium starch glycollate | 4.00 | — | — | — | — | — | — |
| Carbomer Carbopol 981 NF | — | 0.90 | — | — | — | — | — |
| Carbomer Carbopol 1382 NF | — | — | 0.90 | — | — | — | — |
| Carbomer Carbopol 5984 NF | — | — | — | 0.90 | — | — | — |
| Carbomer Carbopol 2984 NF | — | — | — | — | 0.90 | — | — |
| Pemulen | — | — | — | — | — | 0.90 | — |
| Noveon | — | — | — | — | — | — | 0.90 |
| Propylene glycol | 2.00 | 2.0 | | — | — | — | — |
| Fumaric acid | 0.40 | — | — | — | — | — | — |
| Methyl parahydroxybenzoate | 0.16 | 0.16 | 0.16 | 0.16 | 0.16 | 0.16 | 0.16 |
| Propyl parahydroxybenzoate | 0.04 | 0.04 | 0.04 | 0.04 | 0.04 | 0.04 | 0.04 |
| Sodium hydroxide | — | 0.30 | 0.30 | 0.30 | 0.30 | 0.30 | 0.30 |
| Purified water | 83.40 | 86.60 | 86.60 | 86.60 | 86.60 | 86.60 | 86.60 |
| Aspect at preparation | Opaque white odourless gel | | | | | | |
| Aspect at preparation after 3 months at room temperature | Yellowish syrupy liquid having pungent odour | | | | | | |
| Aspect at preparation after 3 months at 40°C temperature | Yellowish syrupy liquid having pungent odour | | | | | | |
| Aspect at preparation after 12 months at room temperature | Yellowish syrupy liquid having ammoniacal, fishy, and pungent odour | | | | | | |
| Aspect at preparation after 12 months at 40°C temperature | Yellowish syrupy liquid having ammoniacal, fishy, and pungent odour | | | | | | |

As it is showed in Table 1 Meldonium dihydrate pharmaceutical compositions has shell life no longer than 3 months after preparation at room temperature.

Therefore unexpected results were achieved by using Carbomer Carbopol 980 NF as thickener, see Table 2 bellow.

### Example

### Meldonium dihydrate topical gel

**Table 2**

| Ingredient | Meldonium dihydrate gel formulation % w/w | | |
|---|---|---|---|
| Meldonium dihydrate | 2.5 | 5.0 | 10.00 |
| Carbomer Carbopol 980 NF | 0.90 | 0.90 | 0.90 |
| Propylene glycol | 2.00 | 2.00 | 2.00 |
| Methyl parahydroxybenzoate | 0.16 | 0.16 | 0.16 |
| Propyl parahydroxybenzoate | 0.04 | 0.04 | 0.04 |
| Sodium hydroxide | 0.30 | 0.30 | 0.30 |
| Purified water | 86.60 | 86.60 | 86.60 |
| Aspect at preparation | Opaque white odourless gel | | |
| Aspect at preparation after 3 months at room temperature | Opaque white odourless gel | | |
| Aspect at preparation after 3 months at 40°C temperature | Opaque white odourless gel | | |
| Aspect at preparation after 12 months at room temperature | Opaque white odourless gel | | |
| Aspect at preparation after 12 months at 40°C temperature | Opaque white odourless gel | | |

| | | | |
|---|---|---|---|
| No traces of separation or decrease in viscosity were observed. | | | |

### Method of preparation Meldonium dihydrate gel

To a solution of carbomer in purified water is added to Meldonium dihydrate while stirring and solution of Meldonium dihydrate-carbomer is obtained. Then aqueous solution of sodium hydroxide is prepared.

The mixture of methyl parahydroxybenzoate, propyl parahydroxybenzoate and propylene glycol is mixed slowly and heated to 60±5°C.The resulting emulsion is allowed to cool to below 25°C while continuously mixing.

A solution of Meldonium dihydrate-carbopol and aqueous solution of sodium hydroxide are next added to the emulsion while continuously mixing.

The gel is homogenized and filled into suitable tubes.

### Industrial applicability

The topical gel of Meldonium dihydrate with stable formulation and increased shelf life is obtained. The topical gel of Meldonium dihydrate can be used for skin irritation treatment

## Claims

1. A topical pharmaceutical composition containing as the active ingredient Meldonium dihydrate to gether with topically acceptable pharmaceutical excipients comprising thickener, humectant and preservatives **characterized in that** as thickener is used Carbopol Carbomer 980.

2. The pharmaceutical composition of claim 1, wherein the amount of Meldonium dihydrate in the composition is from 0.1 % to 40%.

3. The pharmaceutical composition of claim 1, wherein the amount of Meldonium dihydrate 2.5% to 15%.

4. The pharmaceutical composition of claim 1, wherein the amount of Meldonium dihydrate 2.5% to 10%.

5. The composition according to claim 1, wherein humectant is propylene glycol.

6. The composition according to claim 1, wherein preservatives is methyl parahydroxybenzoate and ethyl parahydroxybenzoate.

## Patentansprüche

1. Eine topisch phramzeutische Zusammensetzung, die das aktive Wirkstoff Meldoniumdihydrat enthält, zusammen mit topisch verträglichen pharmazeutischen Hilfsstoffen, bestehend aus Verdickungsmittel, Befeuchtungsmittel und Konservierungsstoffen, **dadurch gekennzeichnet, dass** das Carbopol Carbomer 980 als Verdickungsmittel verwendet wurde.

2. Eine pharmazeutische Zusammensetzung nach Anspruch 1, wobei die Menge an Meldoniumdihydrat in der Zusammensetzung bei 0,1 % bis 40% liegt.

3. Eine pharmazeutische Zusammensetzung nach Anspruch 1, wobei die Menge an Meldoniumdihydrat bei 2,5% bis 15% liegt.

4. Eine pharmazeutische Zusammensetzung nach Anspruch 1, wobei die Menge an Meldoniumdihydrat bei 2,5% bis 10% liegt.

5. Eine Zusammensetzung nach Anspruch 1, wobei das Befeuchtungsmittel Propylenglycol ist.

6. Eine Zusammensetzung nach Anspruch 1, wobei die Konservierungsstoffe Methyl-Parahydroxybenzoate und Ethyl-Parahydroxybenzoate sind.

## Revendications

1. Une composition pharmaceutique topique contenant l'ingrédient actif dihydrate de meldonium avec des excipients pharmaceutiques topiques bien acceptés comprenant un agent épaississant, un agent humectant et des agents de conservation **caractérisés par le fait que** l'agent épaississant utilisé est du Carbopol Carbomer 980.

2. La composition pharmaceutique selon la revendication 1, où la quantité de dihydrate de meldonium se trouvant dans la composition est de 0.1 %à40%.

3. La composition pharmaceutique selon la revendication 1, où la quantité de meldonium dihydrate est de 2.5 % à 15 %.

4. La composition pharmaceutique de la revendication 1, où la quantité de dihydrate de meldonium est de 2.5 % à 10 %.

5. La composition selon la revendication 1, où l'agent humectant est du propylène glycol.

6. La composition selon la revendication 1, où les agents de conservation sont du parahydroxybenzoate de méthyle et de méthyle parahydroxybenzoate.
